# EUROPEAN PATENT SPECIFICATION

(11) **EP 3 206 620 B1**
(45) Date of publication and mention of the grant of the patent: **22.05.2024**
(21) Application number: 15786892.8
(22) Date of filing: 19.10.2015
(51) Int. Cl.: A61B 90/00, A61B 17/34

(54) **SYSTEM FOR PLANNING THE INTRODUCTION OF A NEEDLE IN A PATIENT'S BODY**
SYSTEM ZUR PLANUNG DES EINFÜHRENS EINER NADEL IN DEN KÖRPER EINES PATIENTEN
SYSTÈME POUR PLANIFIER L'INTRODUCTION D'UNE AIGUILLE DANS LE CORPS D'UN PATIENT

(30) Priority: 17.10.2014 EP 14306660
(43) Date of publication of application: 23.08.2017
(73) Proprietor: IMACTIS, 38400 Saint-Martin-d'Hères (FR)
(72) Inventor: CARRAT, Lionel, 38400 Saint Martin d'Heres (FR); LAVALLEE, Stéphane, 38410 St. Martin d'Uriage (FR); BRICAULT, Ivan, 38000 Grenoble (FR); BILLET, Florence, 38000 Grenoble (FR); SIMONOVICI, Patrick-Denis, 38000 Grenoble (FR); LABADIE, Agnès, 38000 Grenoble (FR)
(74) Representative: Regimbeau
(86) International application number: PCT/EP2015/074180
(87) International publication number: WO 2016/059255

(56) References cited:
- EP-A1- 2 777 564
- WO-A1-2010/086374
- WO-A1-2013/055707
- US-A1- 2012 089 008

## Description

### FIELD OF THE INVENTION

The invention relates to a system for planning the introduction of a needle in a patient's body.

### BACKGROUND OF THE INVENTION

Surgical interventions performed in interventional radiology consist in introducing one or more surgical instruments, such as a needles or equivalent, in the body of the patient.

The interventional radiologist uses an imaging system, most likely a Computed Tomography Scan (CT-Scan) or a Cone Beam Computed Tomography (CBCT) or a Magnetic Resonance Imaging system (MRI) to see the organs of the patient and choose the target for the tips and the trajectories to be followed by the needles to reach this target.

In order to help the interventional radiologist to reach the target, a navigation system is necessary. Such systems use a tracking system based on optical, electromagnetic, radiofrequency, inertial, ultrasound or mechanical technology.

The objective of the tracking system is to give the spatial position and orientation in real time of one or more trackers.

Document WO 2010/086374 describes a method for navigating a surgical instrument such as a needle in a 3D medical image of a patient. To that end, the needle is slidingly arranged in a surgical guide to which a tracker is rigidly attached, and a reference marker is attached to the patient's body and localized by the tracking system. Since the reference marker can be detected in the 3D medical image, it is possible to determine the position and orientation of the surgical guide with respect to the 3D medical image. The needle being a linear instrument, its axis is supposed to coincide with the axis of the guide. Hence, even if the needle is not itself tracked, the system allows determining the position and orientation of the needle axis in the 3D medical image.

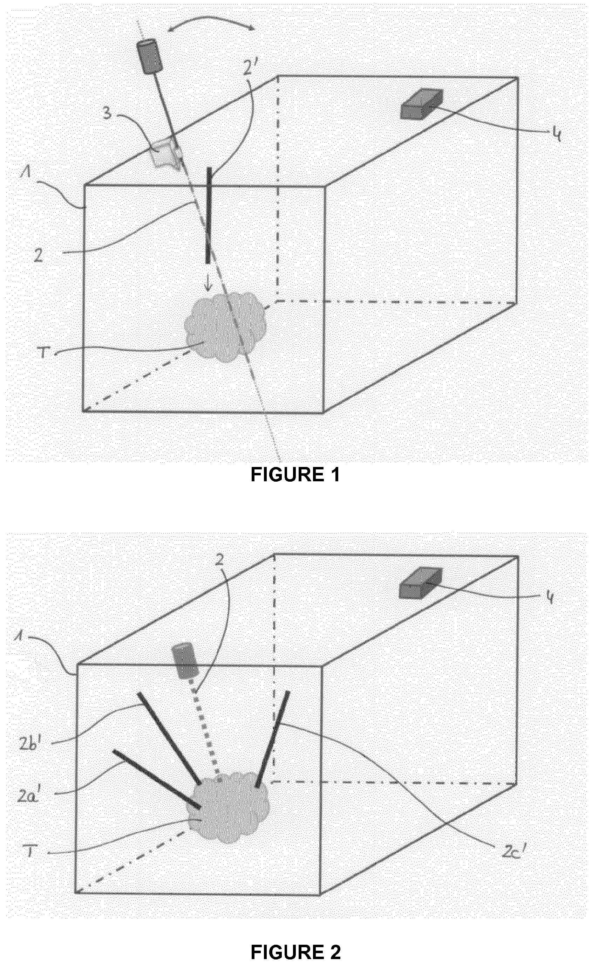

A goal of interventional radiology is to hit a target that moves with the respiration of the patient. As shown in Figure 1, the 3D medical image 1 used by the radiologist to plan the position and orientation of the needle 2 to insert or to push deeper in the body of the patient corresponds to a given time of the respiratory cycle. Moreover, the 3D medical image 1 may contain some patient motion or registration errors. Thus, the virtual needle 2 may not match very well with the detected needle 2' in the 3D medical image and it can move according to the respiration of the patient, as illustrated by the double arrow. The time at which to push again the needle in the body of the patient in order to reach the target T is thus difficult to estimate.

Another goal of interventional radiology is to destroy unwanted cells such as tumours. In order to perform this destruction, more than one needle is sometimes required and these needles have to be placed such that the entire target tumour will be covered and then destroyed. However, as shown in Figure 2, when there is already one or more needles inserted in the body of the patient with their tip in the target T, it is difficult to the radiologist to interpret what happens in three dimensions for all needles.

Document EP 2 777 564 describes a system for assessing the quality of a biopsy of a tissue carried out by a needle.

### BRIEF DESCRIPTION OF THE INVENTION

A goal of the invention is to plan the introduction of a needle in a patient's body using one or more detected needles in a 3D medical image.

According to a first aspect, a needle is already partially inserted in the body of the patient and the invention provides additional guidance to take into account the respiration of the patient to insert deeper the needle in the body of the patient.

According to a second aspect, one or more needles are already inserted in a target and the invention provides additional guidance to plan the insertion of a new needle in this target.

The invention provides a system for planning the introduction of a needle in a patient's body.

### BRIEF DESCRIPTION OF THE DRAWINGS

Other features and advantages of the invention will be apparent from the following description, in connection with the appended drawings wherein:
- Figure 1 illustrates a situation where a navigated needle has not been completely inserted into the patient's body and the virtual needle does not match with the detected needle part already inserted;
- Figure 2 illustrates a situation where a navigated needle has to be inserted into the patient's body wherein several needles have already been inserted in the target;
- Figure 3 illustrates an embodiment of a 2D display of a representation of the virtual needle with respect to the detected needle;
- Figure 4 illustrates an embodiment of a 3D display of a representation of the virtual needle with respect to the detected needle;
- Figure 5 illustrates an embodiment of a 2D display of a representation of the virtual needle with respect to a plurality of detected needles;
- Figure 6 illustrates an embodiment of a 2D display of a representation of the virtual needle with respect to a plurality of detected needles;
- Figure 7 illustrates an embodiment of a 3D display of a representation of the virtual needle with respect to a plurality of detected needles;
- Figure 8 schematically illustrates a system according to the invention.

### DETAILED DESCRIPTION OF EMBODIMENTS OF THE INVENTION

The general context of the method is the planning of the introduction of a needle in a patient's body, the needle being coupled to a needle guide tracked by a navigation system with respect to a 3D medical image of the patient.

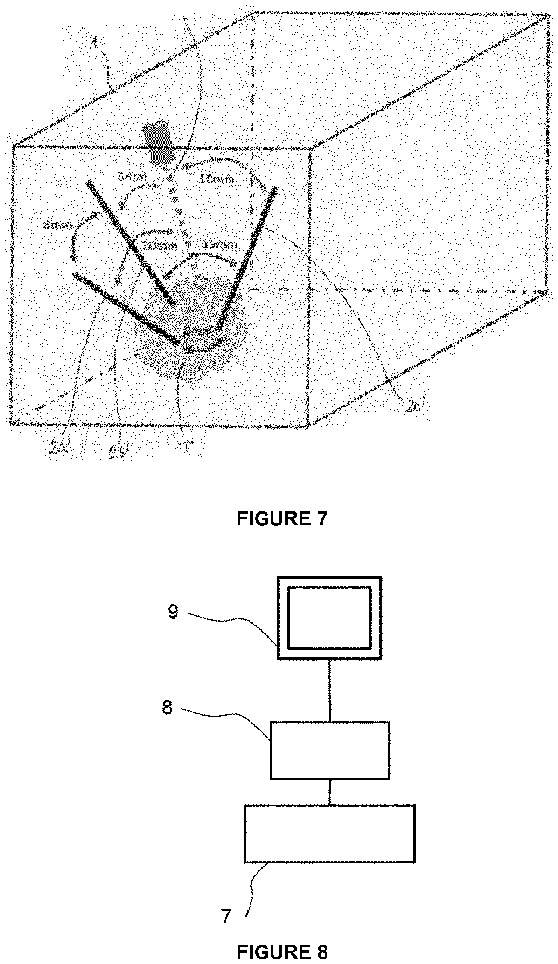

As shown in FIG. 8, the method is implemented by a system comprising a computer including a processor 8 adapted to be coupled to the navigation system 7 to receive navigation data, and a display 9 coupled to the processor 8 and configured for displaying a representation of the virtual needle and a representation of a relative position of the virtual needle with respect to at least one needle detected in the 3D medical image.

Detecting a needle in a 3D image can be achieved using known image processing algorithms that detect line segments as a set of linearly arranged high brightness voxels in a volume of voxels.

Advantageously, the needle guide can be placed on an inserted needle to facilitate its detection. Then, the trace of the needle is detected by using the position of the needle guide in the 3D image given by the navigation system for initializing the search of the detection algorithm. It makes the global process reliable and enables to determine which needle among several needles has to be detected and registered.

According to a first embodiment, the detected needle is the needle that is being navigated and introduced into the patient's body toward a target to be treated by the needle. At this stage, only a part of said needle has been introduced and the needle tip has not reached the target yet. The goal of the user is to place the needle tip in the target.

According to a second embodiment, the detected needle(s) is(are) different from the needle that is introduced into the patient's body and that is tracked in real-time. This corresponds for example to the treatment of a tumor by a plurality of needles, the tips of said needles being intended to be distributed optimally over the tumor volume so as to treat the whole tumor. At this stage, one or more needles have already been placed with their tip in the target and the goal of the user is to place the needle tip in the target taking into account the already inserted needles.

In both cases, the invention proposes to determine a position and orientation of the virtual needle with respect to the 3D image using navigation data of the needle guide; to detect the at least one inserted needle as a trace in the 3D medical image; and to display a representation of the virtual needle and a representation of a relative position of the virtual needle with respect to the at least one detected needle.

The representation of said relative position involves the computation of a 2D or 3D distance between the at least one detected needle and the virtual needle. Such a 2D or 3D distance can be:
(i) a 3D distance from a 3D point of the at least one detected needle to a 3D point of the virtual needle;
(ii) a 3D distance between the line corresponding to the at least one detected needle to a line representing the virtual needle;
(iii) a 3D distance between either a 3D point of the at least one detected needle and a line representing the virtual needle or a 3D point of the virtual needle and a line representing the at least one detected needle;
(iv) 3D distances between either points of the at least one detected needle and a plane containing the virtual needle or points of the virtual needle and a plane containing the detected needle;
(v) a 3D distance between either a line representing a detected needle and a plane containing the virtual needle or a line representing the virtual needle and a plane containing the at least one detected needle;
(vi) a 2D distance between either the virtual needle and a projection of the at least one detected needle in a plane containing the virtual needle or the at least one detected needle and a projection of the virtual needle in a plane containing the at least one detected needle; or
(vii) if several needles are detected, a 3D distance between the tips of the detected needles.

In these distances, the above-mentioned 3D point can advantageously be the center of the active part of the needle, if said needle is a radiofrequency or a cryogeny needle.

The inserted needle can be detected in the 3D medical image by an image processing algorithm.

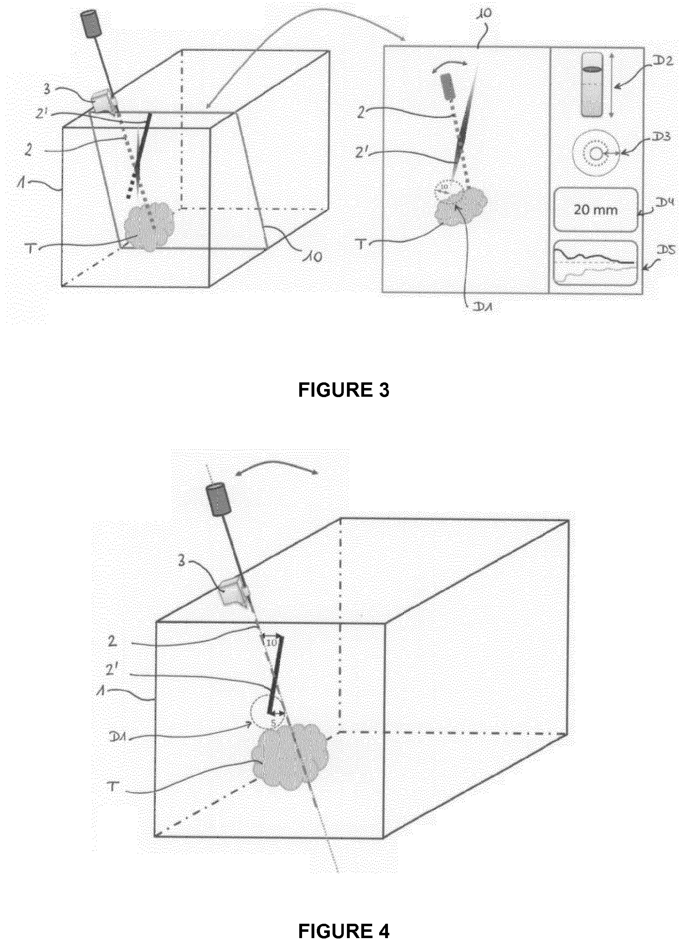

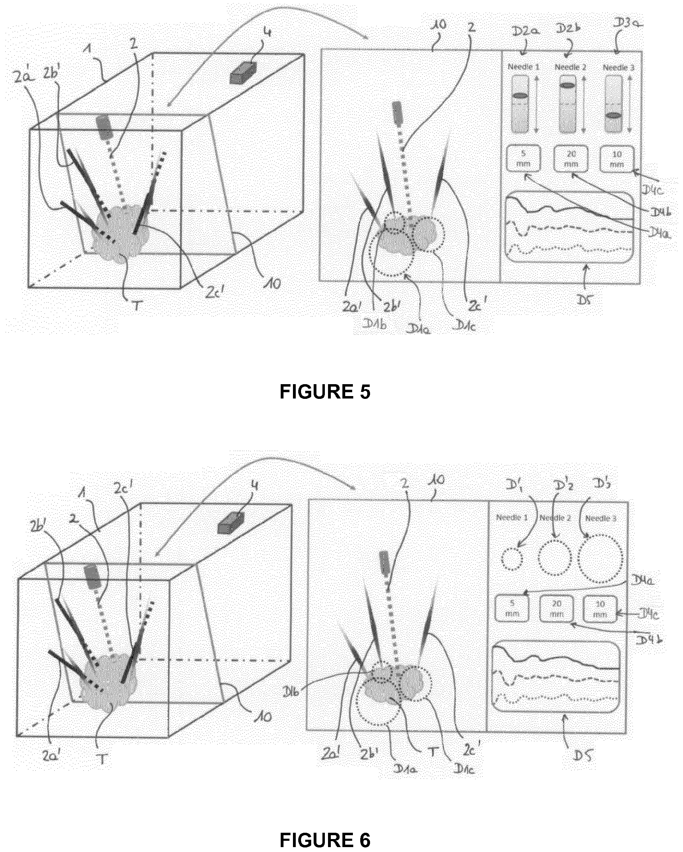

Figures 3 and 4 relate to the first embodiment; Figures 5 to 7 relates to the second embodiment.

Referring to Figure 3 (left), the 3D image 1 contains a trace 2' of the already inserted needle.

This needle is coupled to a needle guide 3 which contains a tracker to be navigated with respect to the 3D image by a navigation system.

The reference 2 represents the virtual needle.

As shown in the right part of Figure 3, a slice 10 according to a plane containing the axis of the virtual needle is displayed with the trace of the detected needle 2' projected in said plane and the target T.

On this slice 10 a circle D1 in dotted line is centred on the projection of the tip of the detected needle 2' in said slice. This circle has a radius which depends on the distance between said tip and the plane containing the axis of the virtual needle (this distance being calculated by the processor); the greater the distance, the larger the radius of this circle. The value of the radius of this circle (10 mm in the illustrated example) may also be displayed.

If the patient breathing generates motions of the partially inserted needle, this distance will vary along the respiratory cycle.

The distance may also be represented according to other formats.

For example, a gauge D2 whose upper and lower extremities are a function of the maximum and the minimum of 3D distance between the virtual and detected needles along the respiratory cycle can be displayed and the current 3D distance is displayed in real time in said gauge.

Another example of representation of the distance is a set D3 of concentric circles, the inner one representing the smaller distance and the outer one representing the larger distance along the respiratory cycle. The circle in dotted lines represents in real time the current distance of the detected needle tip with respect to the plane of the slice 10. Such a representation is a variant of the gauge D2.

Another example of representation of the distance is merely an indication of its numerical value D4.

Another example is a representation of the evolution of the distance with time. The curves D5 show on the one hand the evolution of the distance between the insertion point of the detected needle and the plane of the slice 10 with time, and on the other hand the evolution of the distance between the detected needle tip and the plane of the slice 10 with time, respectively.

Whatever the type of representation displayed, it evolves as the needle guide is moved by the user. The user can thus use the information displayed to check that the orientation and position of the needle guide may allow reaching the target.

With reference to Figure 4, the representation of the distance can also be displayed in the 3D medical image.

For example, a circle D1 whose diameter depends on the distance between the detected needle tip and the virtual needle can be displayed on the tip of said detected needle, along with the value of said distance (here, 5 mm), whereas the value of the distance between the insertion point of the detected needle and the insertion point of the virtual needle (here, 10 mm) is displayed.

The representation of the relative position of the virtual needle with respect to the at least one detected needle can also be based on a set of transparency levels of the detected needle in 3D or of the projection of the detected needle in the plane containing the virtual needle (see Figure 3). For example, the representation of the detected needle is all the more opaque that the needle is close to the virtual needle.

The representation of the relative position of the virtual needle with respect to the at least one detected needle can also be based on a set of thickness levels of the detected needle in 3D or of the projection of the detected needle in the plane containing the virtual needle (see Figure 3). For example, the representation of the detected needle is all the more thick that the needle is close to the virtual needle. This explains the lozenge shape of the projection of the detected needle in Figure 3.

Of course, two or more of these various representations can be combined and displayed together. In addition, the skilled person may select any other way of representing the distance without departing from the scope of the invention.

Advantageously, the respiration of the patient can be taken into account in order to determine an optimal instant for the user to further push the needle into the patient's body.

To that end, an instant of the respiratory cycle of the patient at which the virtual needle is closest to the detected needle is determined. The virtual position of the needle at said instant is then registered to the detected needle. Then, the representation of the virtual needle with respect to the detected needle is displayed again.

Besides, the determination of said instant of the respiratory cycle of the patient at which the virtual needle is closest to the detected needle can be used to provide an information to the user to push the needle into the patient's body at said instant, since this will give the best chance to reach the target. Indeed when both virtual and real needle coincide, it is considered that the patient breathing is at the same cycle position than it was when the 3D image was acquired. Therefore this method offers a virtual synchronization of time between the 3D image and the navigation.

According to a second embodiment illustrated in Figures 5 to 7, the 3D image contains the trace of at least one detected needle which is different from the needle that is introduced into the patient's body and which has already been inserted into the patient's body and has reached the target.

In the embodiments of Figures 5 to 7, three needles 2a', 2b' and 2c' are detected in the 3D image 1 and have their tip in the target T. The needle to be additionally inserted in the target is coupled to a needle guide 3 which contains a tracker to be navigated with respect to the 3D image by a navigation system.

The reference 2 represents the virtual needle.

As shown in the right part of Figures 5 and 6, a slice 10 according to a plane containing the axis of the virtual needle is displayed with the trace of the detected needles 2a', 2b', 2c' projected in said plane and the target T.

The 2D or 3D distance between the virtual needle and each detected needle is calculated as explained above.

The indication of the distance between the virtual needle and each detected needle is represented in the similar way as already described with reference to Figures 3 and 4. These representations are thus not described again in detail.

For example, as shown in Figure 5 (left), three gauges D2a, D2b, D2c are displayed.

Alternatively or in combination with the above representation, a circle D1a, D1b, D1c is centered on the projection of each respective detected needle, the radius of each circle depending on the distance between said detected needle and the virtual needle.

Alternatively or in combination with at least one of the above representations, the numerical value D4a, D4b, D4c of the distance between the virtual needle and each respective detected needle is displayed.

Alternatively or in combination with at least one of the above representations, curves D5 illustrated the evolution of the distance with time is displayed.

Figure 6 illustrates an embodiment which is substantially similar to the one of Figure 5, apart from the fact that the indication of the distance between the virtual needle and each detected needle is represented by a respective circle D1', D2', D3' whose radius depends on said distance.

As shown in Figure 7, the representation of the distance can also be displayed in the 3D medical image 1. Numerical values of the distance between each detected needle and the virtual needle - and/or between two detected needles - can be displayed.

As already described above, a set of transparency levels and/or of thickness levels can also be applied to each detected needle.

As shown in Figures 3 to 7, it is also useful to display a 3D representation of the complete scene containing the previously detected needles as 3D line segments, as well as the navigated needle in real-time as another line segment, and also the target (e.g. tumor) as a surface, with indications of the relative 3D distances in addition.

With this displayed information, the user is capable of determining the position and orientation needed for the navigated needle in order to distribute the needles optimally over the target.

## Claims

1. System for planning the introduction of a needle in a patient's body, comprising:
- a needle guide (3) adapted to be coupled to a needle (2), wherein the needle guide (3) is selected from:
(i) a guide in which the needle is adapted to be slidingly arranged, said guide comprising a tracker configured to be tracked by the navigation system;
(ii) a guide adapted to be rigidly attached to the needle, said guide comprising a tracker configured to be tracked by the navigation system; and
(iii) a tracker configured to be tracked by the navigation system, said tracker being adapted to be arranged inside the needle.;
- a navigation system configured for tracking the needle guide (3) with respect to a 3D medical image of the patient;
- a processor configured for:
- receiving navigation data of the needle guide (3) from the navigation system,
- determining a virtual needle having a position and an orientation with respect to the 3D image (1) that are computed based on said navigation data of the needle guide (3),
- detecting at least one inserted needle (2'; 2a', 2b', 2c') as a trace in the 3D medical image (1),
- computing a distance between the virtual needle (2) and the detected needle (2'; 2a', 2b', 2c'), and
- determining a representation of said computed distance;
- a display coupled to the processor for displaying a representation of the virtual needle (2) and said determined representation of the computed distance between the virtual needle and the at least one detected needle (2'; 2a', 2b', 2c').

2. System according to claim 1, wherein the processor is configured to implement an image processing algorithm to detect the at least one inserted needle.

3. System according to claim 1, wherein the processor is configured to detect the at least one inserted needle by using the navigation system for an initialization of the detected needle position and orientation.

4. System according to any of claims 1 to 3, wherein the trace detected in the 3D medical image is a trace of a part of the navigated needle that has already been inserted into the patient's body.

5. System according to claim 4, wherein the processor is configured for:
- determining an instant of the respiratory cycle of the patient at which the virtual needle is closest to the detected needle; and
- registering the virtual position of the needle at said instant to the detected needle;
and wherein the display is configured for displaying again a representation of the distance between the virtual needle and the detected needle.

6. System according to claim 4 or claim 5, wherein the processor is configured for determining an instant of the respiratory cycle of the patient at which the virtual needle (2) is closest to the detected needle (2') and providing an information to a user to push the needle into the patient's body at said instant.

7. System according to any of claims 4 to 6, wherein the representation of the computed distance between the virtual needle and the detected needle comprises an indication of at least one 2D or 3D distance selected from:
(i) a 3D distance from a 3D point of the detected needle to a 3D point of the virtual needle;
(ii) a 3D distance between a line representing the detected needle to a line representing the virtual needle;
(iii) a 3D distance between either a 3D point of the detected needle and a line representing the virtual needle or a 3D point of the virtual needle and a line representing the detected needle;
(iv) 3D distances between either points of the detected needle and a plane containing the virtual needle or points of the virtual needle and a plane containing the detected needle;
(v) a 3D distance between either a line representing the detected needle and a plane containing the virtual needle or a line representing the virtual needle and a plane containing the detected needle; and
(vi) a 2D distance between either the virtual needle and a projection of the detected needle in a plane containing the virtual needle or the detected needle and a projection of the virtual needle in a plane containing the detected needle.

8. System according to claim 7, wherein the display is configured for displaying said at least one 2D or 3D distance in at least one of the following formats:
- a number (D4) corresponding to the numerical value of said distance;
- a gauge (D2) with extremities corresponding to a function of the maximum and the minimum of these 3D distances along the respiratory cycle;
- a curve (D3) showing the evolution of the distance with time;
- a set of transparency levels of the detected needle in 3D or of a projection of the detected needle in the plane containing the virtual needle;
- a set of thickness levels of the detected needle in 3D or of the projection of the detected needle in the plane containing the virtual needle;
- a circle (D1) displayed on a plane containing the virtual needle, centered on the projection of the tip of the detected needles on the given plane and which radius is a function of said indicated 2D or 3D distance.

9. System according to any of claims 1 to 3, wherein the trace detected in the 3D medical image is a trace of a needle (2a', 2b', 2c') distinct from the virtual needle (2) and that has already been inserted into the patient's body.

10. System according to claim 9, wherein the representation of the computed distance between the virtual needle and the at least one detected needle comprises an indication of at least a 2D or 3D distance selected from:
(i) a 3D distance from a 3D point of a detected needle to a respective 3D point of the virtual needle;
(ii) a 3D distance between the line corresponding to a detected needle to a line representing the virtual needle;
(iii) a 3D distance between either a 3D point of a detected needle and a line representing the virtual needle or a 3D point of the virtual needle and a line representing a detected needle;
(iv) 3D distances between either points of a detected needle and a plane containing the virtual needle or points of a virtual needle and a plane containing a detected needle;
(v) a 3D distance between either a line representing a detected needle and a plane containing the virtual needle or a line representing the virtual needle and a plane containing one detected needle;
(vi) a 2D distance between either the virtual needle and a projection of a detected needle in a plane containing the virtual needle or a detected needle and a projection of the virtual needle in a plane containing said detected needle; and
(vii) a 3D distance between the tips of the detected needles.

11. System according to claim 10, wherein the display is configured to display said at least one 2D or 3D distance in at least one of the following formats:
- a number corresponding to the numerical value of said distance;
- a curve showing the evolution of the distance with time;
- a set of transparency levels of the detected needles in 3D or of the projection of the detected needles in a plane containing the virtual needle;
- a set of thickness levels of the detected needles in 3D or of the projection of the detected needles in a plane containing the virtual needle;
- a sphere centered on the tip of the detected needles and whose radius is a function of said distance;
- a circle displayed on a plane containing the virtual needle, centered on the projection of the tip of the detected needles on the given plane and which radius is a function of said distance.

12. System according to one of claims 9 to 11, wherein the processor is configured for determining an instant of the respiratory cycle of the patient where the virtual needle is at an optimal position relative to a target and to each detected needle and providing an information to a user to push the needle into the patient's body at said instant.

13. Computer program product comprising computer-readable instructions which, when loaded and executed on the processor of a system according to one of claims 1 to 12, perform the steps of:
- determining a virtual position and orientation of the needle with respect to the 3D image using navigation data of the needle guide;
- detecting at least one inserted needle as a trace in the 3D medical image;
- computing a distance between the virtual needle and the detected needle;
- determining a representation of said computed distance;
- displaying a representation of the virtual needle (2) and said determined representation of the computed distance between the virtual needle and the at least one detected needle (2'; 2a', 2b', 2c').

## Patentansprüche

1. System zur Planung der Einführung einer Nadel in den Körper eines Patienten, bestehend aus:
- einer Nadelführung (3), die so angepasst ist, dass sie mit einer Nadel (2) gekoppelt werden kann, wobei die Nadelführung (3) ausgewählt wird aus:
(i) einer Führung, in der die Nadel so angepasst ist, dass sie gleitend angeordnet ist, wobei diese Führung einen Tracker umfasst, der so konfiguriert ist, dass er vom Navigationssystem verfolgt werden kann;
(ii) einer Führung, die so angepasst ist, dass sie starr an der Nadel befestigt ist, wobei diese Führung einen Tracker umfasst, der so konfiguriert ist, dass er vom Navigationssystem verfolgt werden kann; und
(iii) einem Tracker, der so konfiguriert ist, dass er vom Navigationssystem verfolgt werden kann, wobei dieser Tracker so angepasst ist, dass er im Inneren der Nadel platziert wird;
- einem Navigationssystem, das so konfiguriert ist, dass es die Nadelführung (3) in Bezug auf ein medizinisches 3D-Bild des Patienten verfolgt;
- einem Prozessor, der so konfiguriert ist, dass er:
- Navigationsdaten der Nadelführung (3) vom Navigationssystem empfängt,
- eine virtuelle Nadel mit einer Position und einer Ausrichtung in Bezug auf das 3D-Bild (1) bestimmt, die anhand dieser Navigationsdaten der Nadelführung (3) berechnet werden,
- mindestens eine eingeführte Nadel (2'; 2a', 2b', 2c') als Spur im medizinischen 3D-Bild (1) bestimmt,
- einen Abstand zwischen der virtuellen Nadel (2) und der erkannten Nadel (2'; 2a', 2b', 2c') berechnet, und
- eine Darstellung dieses berechneten Abstands bestimmt;
- einem mit dem Prozessor gekoppelten Display zur Anzeige einer Darstellung der virtuellen Nadel (2) und dieser bestimmten Darstellung des berechneten Abstands zwischen der virtuellen Nadel und der mindstens einen erkannten Nadel (2'; 2a', 2b', 2c').

2. System nach Anspruch 1, wobei der Prozessor so konfiguriert ist, dass er einen Bildverarbeitungsalgorithmus beinhaltet, um die mindstens eine eingeführte Nadel zu erkennen.

3. System nach Anspruch 1, wobei der Prozessor so konfiguriert ist, dass er die mindstens eine eingeführte Nadel erkennt, indem er das Navigationssystem zur Initialisierung der erkannten Nadelposition und -ausrichtung verwendet.

4. System nach einem der Ansprüche 1 bis 3, wobei es sich bei der im medizinischen 3D-Bild erkannten Spur um eine Spur eines Teils der navigierten Nadel handelt, die bereits in den Körper des Patienten eingeführt wurde.

5. System nach Anspruch 4, wobei der Prozessor so konfiguriert ist, dass er:
- einen Zeitpunkt im Atemzyklus des Patienten bestimmt, an dem die virtuelle Nadel der erkannten Nadel am nächsten ist; und
- die virtuelle Position der Nadel zu diesem Zeitpunkt an der erkannten Nadel erfasst;
und wobei das Display so konfiguriert ist, dass es erneut eine Darstellung des Abstands zwischen der virtuellen Nadel und der erkannten Nadel anzeigt.

6. System nach Anspruch 4 oder Anspruch 5, wobei der Prozessor so konfiguriert ist, dass er einen Zeitpunkt im Atemzyklus des Patienten bestimmt, an dem die virtuelle Nadel (2) der erkannten Nadel (2') am nächsten ist, und dem Benutzer eine Information liefert, die Nadel zu diesem Zeitpunkt in den Körper des Patienten zu drücken.

7. System nach einem der Ansprüche 4 bis 6, wobei die Darstellung des berechneten Abstands zwischen der virtuellen Nadel und der erkannten Nadel eine Angabe von mindestens einem 2D- oder 3D-Abstand umfasst, der ausgewählt wird aus:
(i) einem 3D-Abstand von einem 3D-Punkt der erkannten Nadel zu einem 3D-Punkt der virtuellen Nadel;
(ii) einem 3D-Abstand zwischen einer Linie, die die erkannte Nadel darstellt, und einer Linie, die die virtuelle Nadel darstellt;
(iii) einem 3D-Abstand zwischen entweder einem 3D-Punkt der erkannten Nadel und einer Linie, die die virtuelle Nadel darstellt, oder einem 3D-Punkt der virtuellen Nadel und einer Linie, die die erkannte Nadel darstellt;
(iv) 3D-Abständen zwischen entweder Punkten der erkannten Nadel und einer Ebene, die die virtuelle Nadel enthält, oder Punkten der virtuellen Nadel und einer Ebene, die die erkannte Nadel enthält;
(v) einem 3D-Abstand zwischen entweder einer Linie, die die erkannte Nadel darstellt, und einer Ebene, die die virtuelle Nadel enthält, oder einer Linie, die die virtuelle Nadel darstellt, und einer Ebene, die die erkannte Nadel enthält; und
(vi) einem 2D-Abstand zwischen entweder der virtuellen Nadel und einer Projektion der erkannten Nadel in einer Ebene, die die virtuelle Nadel enthält, oder der erkannten Nadel und einer Projektion der virtuellen Nadel in einer Ebene, die die erkannte Nadel enthält.

8. System nach Anspruch 7, wobei das Display so konfiguriert ist, dass es diesen mindestens einen 2D- oder 3D-Abstand in mindestens einem der folgenden Formate anzeigt:
- eine Zahl (D4), die dem numerischen Wert dieses Abstands entspricht;
- ein Sensor (D2) mit Extremitäten, die einer Funktion der maximalen und minimalen dieser 3D-Abstände entlang des Atemzyklus entsprechen;
- ein Sensor (D2) mit Extremitäten, die einer Funktion der maximalen und minimalen dieser 3D-Abstände entlang des Atemzyklus entsprechen;
- eine Kurve (D3), die die Entwicklung des Abstands im Zeitverlauf zeigt;
- eine Reihe von Transparenzstufen der erkannten Nadel in 3D oder einer Projektion der erkannten Nadel in der Ebene, die die virtuelle Nadel enthält;
- eine Reihe von Stärkestufen der erkannten Nadel in 3D oder der Projektion der erkannten Nadel in der Ebene, die die virtuelle Nadel enthält;
- ein Kreis (D1), der auf einer Ebene angezeigt wird, die die virtuelle Nadel enthält, zentriert auf der Projektion der Spitze der erkannten Nadeln auf der jeweiligen Ebene, und bei dessen Radius es sich um eine Funktion dieses angegebenen 2D- oder 3D-Abstands handelt.

9. System nach einem der Ansprüche 1 bis 3, wobei es sich bei der im medizinischen 3D-Bild erkannten Spur um eine Spur einer Nadel (2a', 2b', 2c') handelt, die sich von der virtuellen Nadel (2) unterscheidet und die bereits in den Körper des Patienten eingeführt wurde.

10. System nach Anspruch 9, wobei die Darstellung des berechneten Abstands zwischen der virtuellen Nadel und der mindestens einen erkannten Nadel eine Angabe von mindestens einem 2D- oder 3D-Abstand umfasst, der ausgewählt wird aus:
(i) einem 3D-Abstand von einem 3D-Punkt einer erkannten Nadel zu einem entsprechenden 3D-Punkt der virtuellen Nadel;
(ii) einem 3D-Abstand zwischen der Linie, die einer erkannten Nadel entspricht, und einer Linie, die die virtuelle Nadel darstellt;
(iii) einem 3D-Abstand zwischen entweder einem 3D-Punkt einer erkannten Nadel und einer Linie, die die virtuelle Nadel darstellt, oder einem 3D-Punkt der virtuellen Nadel und einer Linie, die eine erkannte Nadel darstellt;
(iv) 3D-Abständen zwischen entweder Punkten einer erkannten Nadel und einer Ebene, die die virtuelle Nadel enthält, oder Punkten einer virtuellen Nadel und einer Ebene, die eine erkannte Nadel enthält;
(v) einem 3D-Abstand zwischen entweder einer Linie, die eine erkannte Nadel darstellt, und einer Ebene, die die virtuelle Nadel enthält, oder einer Linie, die die virtuelle Nadel darstellt, und einer Ebene, die eine erkannte Nadel enthält;
(vi) einem 2D-Abstand zwischen entweder der virtuellen Nadel und einer Projektion einer erkannten Nadel in einer Ebene, die die virtuelle Nadel enthält, oder einer erkannten Nadel und einer Projektion der virtuellen Nadel in einer Ebene, die diese erkannte Nadel enthält; und
(vii) einem 3D-Abstand zwischen den Spitzen der erkannten Nadeln.

11. System nach Anspruch 10, wobei das Display so konfiguriert ist, dass es diesen mindestens einen 2D- oder 3D-Abstand in mindestens einem der folgenden Formate anzeigt:
- eine Zahl, die dem numerischen Wert dieses Abstands entspricht;
- eine Kurve, die die Entwicklung des Abstands im Zeitverlauf zeigt;
- eine Reihe von Transparenzstufen der erkannten Nadeln in 3D oder der Projektion der erkannten Nadeln in einer Ebene, die die virtuelle Nadel enthält;
- eine Reihe von Stärkestufen der erkannten Nadeln in 3D oder der Projektion der erkannten Nadeln in einer Ebene, die die virtuelle Nadel enthält;
- eine Sphäre, zentriert auf der Spitze der erkannten Nadeln, und bei deren Radius es sich um eine Funktion dieses Abstands handelt;
- ein Kreis, der auf einer Ebene angezeigt wird, die die virtuelle Nadel enthält, zentriert auf der Projektion der Spitze der erkannten Nadeln auf der jeweiligen Ebene, und bei dessen Radius es sich um eine Funktion dieses Abstands handelt.

12. System nach einem der Ansprüche 9 bis 11, wobei der Prozessor so konfiguriert ist, dass er einen Zeitpunkt im Atemzyklus des Patienten bestimmt, an dem sich die virtuelle Nadel in einer optimalen Position relativ zu einem Ziel und zu jeder erkannten Nadel befindet, und dem Benutzer eine Information liefert, die Nadel zu diesem Zeitpunkt in den Körper des Patienten zu drücken.

13. Computerprogrammprodukt, das computerlesbare Anweisungen umfasst, die, wenn sie auf den Prozessor eines Systems nach einem der Ansprüche 1 bis 12 geladen und ausgeführt werden, die Schritte ausführen, um:
- anhand von Navigationsdaten der Nadelführung eine virtuelle Position und Ausrichtung der Nadel in Bezug auf das 3D-Bild zu bestimmen;
- mindestens eine eingeführte Nadel als Spur im medizinischen 3D-Bild zu bestimmen;
- einen Abstand zwischen der virtuellen Nadel und der erkannten Nadel zu berechnen; - eine Darstellung dieses berechneten Abstands zu bestimmen;
- eine Darstellung der virtuellen Nadel (2) und dieser bestimmten Darstellung des berechneten Abstands zwischen der virtuellen Nadel und der mindstens einen erkannten Nadel (2'; 2a', 2b', 2c') anzuzeigen.

## Revendications

1. Système de planification de l'introduction d'une aiguille dans le corps d'un patient, comprenant :
- un guide-aiguille (3) adapté pour être couplé à une aiguille (2), dans lequel le guide-aiguille (3) est choisi parmi :
(i) un guide dans lequel l'aiguille est adaptée pour être disposée de manière coulissante, ledit guide comprenant un traceur configuré pour être suivi par le système de navigation ;
(ii) un guide adapté pour être fixé rigidement à l'aiguille, ledit guide comprenant un traceur configuré pour être suivi par le système de navigation ; et
(iii) un traceur configuré pour être suivi par le système de navigation, ledit traceur étant adapté pour être disposé à l'intérieur de l'aiguille. ;
- un système de navigation configuré pour suivre le guide-aiguille (3) par rapport à une image médicale 3D du patient ;
- un processeur configuré pour :
- recevoir du système de navigation les données de navigation du guide-aiguille (3),
- déterminer une aiguille virtuelle ayant une position et une orientation par rapport à l'image 3D (1) qui sont calculées sur la base des données de navigation du guide-aiguille (3),
- détecter au moins une aiguille insérée (2' ; 2a', 2b', 2c') sous forme de trace dans l'image médicale 3D (1),
- calculer une distance entre l'aiguille virtuelle (2) et l'aiguille détectée (2' ; 2a', 2b', 2c'), et
- déterminer une représentation de la distance calculée ;
- un écran couplé au processeur pour afficher une représentation de l'aiguille virtuelle (2) et ladite représentation déterminée de la distance calculée entre l'aiguille virtuelle et l'au moins une aiguille détectée (2' ; 2a', 2b', 2c').

2. Système selon la revendication 1, dans lequel le processeur est configuré pour mettre en oeuvre un algorithme de traitement d'image afin de détecter l'au moins une aiguille insérée.

3. Système selon la revendication 1, dans lequel le processeur est configuré pour détecter l'au moins une aiguille insérée en utilisant le système de navigation pour une initialisation de la position et de l'orientation de l'aiguille détectée.

4. Système selon l'une des revendications 1 à 3, dans lequel la trace détectée dans l'image médicale 3D est une trace d'une partie de l'aiguille naviguée qui a déjà été insérée dans le corps du patient.

5. Système selon la revendication 4, dans lequel le processeur est configuré pour :
- déterminer un moment du cycle respiratoire du patient où l'aiguille virtuelle est la plus proche de l'aiguille détectée ; et
- le recalage de la position virtuelle de l'aiguille à cet instant avec l'aiguille détectée ;
et dans lequel l'écran est configuré pour afficher à nouveau une représentation de la distance entre l'aiguille virtuelle et l'aiguille détectée.

6. Système selon la revendication 4 ou la revendication 5, dans lequel le processeur est configuré pour déterminer un instant du cycle respiratoire du patient auquel l'aiguille virtuelle (2) est la plus proche de l'aiguille détectée (2') et pour fournir une information à un utilisateur afin qu'il enfonce l'aiguille dans le corps du patient à cet instant.

7. Système selon l'une des revendications 4 à 6, dans lequel la représentation de la distance calculée entre l'aiguille virtuelle et l'aiguille détectée comprend une indication d'au moins une distance 2D ou 3D choisie parmi :
(i) une distance 3D entre un point 3D de l'aiguille détectée et un point 3D de l'aiguille virtuelle ;
(ii) une distance 3D entre une ligne représentant l'aiguille détectée et une ligne représentant l'aiguille virtuelle ;
(iii) une distance 3D entre un point 3D de l'aiguille détectée et une ligne représentant l'aiguille virtuelle ou un point 3D de l'aiguille virtuelle et une ligne représentant l'aiguille détectée ;
(iv) les distances 3D entre les points de l'aiguille détectée et un plan contenant l'aiguille virtuelle ou entre les points de l'aiguille virtuelle et un plan contenant l'aiguille détectée ;
(v) une distance 3D entre une ligne représentant l'aiguille détectée et un plan contenant l'aiguille virtuelle ou une ligne représentant l'aiguille virtuelle et un plan contenant l'aiguille détectée ; et
(vi) une distance 2D entre l'aiguille virtuelle et une projection de l'aiguille détectée dans un plan contenant l'aiguille virtuelle ou l'aiguille détectée et une projection de l'aiguille virtuelle dans un plan contenant l'aiguille détectée.

8. Système selon la revendication 7, dans lequel l'écran est configuré pour afficher ladite au moins une distance 2D ou 3D dans au moins l'un des formats suivants
- un nombre (D4) correspondant à la valeur numérique de cette distance ;
- une jauge (D2) dont les extrémités correspondent à une fonction du maximum et du minimum de ces distances 3D au cours du cycle respiratoire ;
- une courbe (D3) montrant l'évolution de la distance dans le temps ;
- un ensemble de niveaux de transparence de l'aiguille détectée en 3D ou d'une projection de l'aiguille détectée dans le plan contenant l'aiguille virtuelle ;
- un ensemble de niveaux d'épaisseur de l'aiguille détectée en 3D ou de la projection de l'aiguille détectée dans le plan contenant l'aiguille virtuelle ;
- un cercle (D1) affiché sur un plan contenant l'aiguille virtuelle, centré sur la projection de la pointe des aiguilles détectées sur le plan donné et dont le rayon est fonction de ladite distance 2D ou 3D indiquée.

9. Système selon l'une des revendications 1 à 3, dans lequel la trace détectée dans l'image médicale 3D est la trace d'une aiguille (2a', 2b', 2c') distincte de l'aiguille virtuelle (2) et qui a déjà été insérée dans le corps du patient.

10. Système selon la revendication 9, dans lequel la représentation de la distance calculée entre l'aiguille virtuelle et l'au moins une aiguille détectée comprend une indication d'au moins une distance 2D ou 3D sélectionnée parmi :
(i) une distance 3D entre un point 3D d'une aiguille détectée et un point 3D respectif de l'aiguille virtuelle ;
(ii) une distance 3D entre la ligne correspondant à une aiguille détectée et une ligne représentant l'aiguille virtuelle ;
(iii) une distance 3D entre un point 3D d'une aiguille détectée et une ligne représentant l'aiguille virtuelle ou un point 3D de l'aiguille virtuelle et une ligne représentant une aiguille détectée ;
(iv) les distances en 3D entre les points d'une aiguille détectée et un plan contenant l'aiguille virtuelle ou les points d'une aiguille virtuelle et un plan contenant une aiguille détectée ;
(v) une distance 3D entre une ligne représentant une aiguille détectée et un plan contenant l'aiguille virtuelle ou une ligne représentant l'aiguille virtuelle et un plan contenant une aiguille détectée ;
(vi) une distance 2D entre l'aiguille virtuelle et une projection d'une aiguille détectée dans un plan contenant l'aiguille virtuelle ou une aiguille détectée et une projection de l'aiguille virtuelle dans un plan contenant ladite aiguille détectée ; et
(vii) une distance en 3D entre les extrémités des aiguilles détectées.

11. Système selon la revendication 10, dans lequel l'écran est configuré pour afficher ladite au moins une distance 2D ou 3D dans au moins l'un des formats suivants
- un nombre correspondant à la valeur numérique de cette distance ;
- une courbe montrant l'évolution de la distance en fonction du temps ;
- un ensemble de niveaux de transparence des aiguilles détectées en 3D ou de la projection des aiguilles détectées dans un plan contenant l'aiguille virtuelle ;
- un ensemble de niveaux d'épaisseur des aiguilles détectées en 3D ou de la projection des aiguilles détectées dans un plan contenant l'aiguille virtuelle ;
- une sphère centrée sur la pointe des aiguilles détectées et dont le rayon est fonction de ladite distance ;
- un cercle affiché sur un plan contenant l'aiguille virtuelle, centré sur la projection de la pointe des aiguilles détectées sur le plan donné et dont le rayon est fonction de ladite distance.

12. Système selon l'une des revendications 9 à 11, dans lequel le processeur est configuré pour déterminer un instant du cycle respiratoire du patient où l'aiguille virtuelle est à une position optimale par rapport à une cible et à chaque aiguille détectée et pour fournir une information à un utilisateur afin qu'il enfonce l'aiguille dans le corps du patient à cet instant.

13. Produit programme d'ordinateur comprenant des instructions lisibles par ordinateur qui, lorsqu'elles sont chargées et exécutées sur le processeur d'un système selon l'une des revendications 1 à 12, exécutent les étapes suivantes :
- déterminer une position et une orientation virtuelles de l'aiguille par rapport à l'image 3D en utilisant les données de navigation du guide de l'aiguille ;
- détecter au moins une aiguille insérée sous forme de trace dans l'image médicale 3D ;
- calculer la distance entre l'aiguille virtuelle et l'aiguille détectée ;
- déterminer une représentation de ladite distance calculée ;
- afficher une représentation de l'aiguille virtuelle (2) et ladite représentation déterminée de la distance calculée entre l'aiguille virtuelle et l'au moins une aiguille détectée (2' ; 2a', 2b', 2c').
